# EUROPEAN PATENT SPECIFICATION

(11) **EP 3 620 108 B1**
(45) Date of publication and mention of the grant of the patent: **21.04.2021**
(21) Application number: 18192456.4
(22) Date of filing: 04.09.2018
(51) Int. Cl.: A61B 5/0404, A61B 5/0428, A61B 5/0488, A61B 5/0496, A61B 5/053, A61B 5/00

(54) **A SYSTEM AND A METHOD FOR ACQUIRING AN ELECTRICAL SIGNAL AND A WEARABLE DEVICE**
SYSTEM UND VERFAHREN ZUR ERFASSUNG EINES ELEKTRISCHEN SIGNALS UND EINE WEARABLE-VORRICHTUNG
SYSTÈME ET PROCÉDÉ PERMETTANT L'ACQUISITION D'UN SIGNAL ÉLECTRIQUE ET DISPOSITIF VESTIMENTAIRE

(43) Date of publication of application: 11.03.2020
(73) Proprietor: Stichting IMEC Nederland, 5656 AE Eindhoven (NL)
(72) Inventor: ZALIASL, Samira, 3001 Leuven (BE); STANZIONE, Stefano, 3001 Leuven (BE); PATKI, Shrishail, 3001 Leuven (BE)
(74) Representative: AWA Sweden AB

(56) References cited:
- WO-A1-2018/093181
- US-A1- 2007 239 220
- US-A1- 2011 288 605

## Description

### Technical field

The present inventive concept relates to a system and a method for acquiring of an electrical signal. In particular, the present inventive concept relates to acquiring of a bio-electrical signal, such as an electrocardiogram signal, and the present inventive concept also relates to a wearable device for acquiring the electrical signal from a subject wearing the device.

### Background

Sensing of electrical signals from living beings may be useful for many different purposes, e.g. in determining or monitoring of a medical condition of the living being. The electrical signals may be any bio-electrical signal, such as a signal naturally occurring in the living being, e.g. an electrocardiogram (ECG) signal, or an induced signal, such as a bio-impedance signal as induced based on a stimulation signal provided to a body of the living being.

In sensing of bio-electrical signals using a wearable device, one of the worst problems to solve is the presence of artifacts in the signal. Artifacts may be due to multiple time-variant phenomena, such as motion of a subject wearing the device, change of electrode properties, change of contact impedance, movements of internal organs. Such phenomena are all factors that can affect a quality of a detected signal.

In order to address artifacts, there is a lot of interest in building up systems with a variety of electrodes, hoping to be capable of recognizing the pair of electrodes which carry the cleanest possible signal.

US 6,217,525 discloses an electrocardiogram arrangement having a plurality of lead channels, in which at least two lead channels are assigned an evaluation unit which evaluates the ECG quality, classifies the results and locates the QRS complexes. For the QRS complex location, a central logic unit selects the lead channel which has the highest ECG quality as the dominant lead channel. When special events occur in the dominant lead channel, other lead channels are accessed as appropriate.

However, use of a plurality of electrodes implies that a complex system is provided. A readout circuitry for each pair of electrodes may be needed and the number of readout circuitries grows fast with the number of electrodes of the system. Additionally, the readout circuitries will consume power, such that a total power consumption of the system will be high. Therefore, there is a need of controlling power consumption while allowing a system using a plurality of electrodes for enabling acquiring of a signal of high quality.

### Summary

An objective of the present inventive concept is to provide a sensing system which enables high quality acquisition of an electrical signal with a limited power consumption of the sensing system. A particular objective of the present inventive concept is to provide sensing of a bio-electrical signal, which sensing may be affected by changing sensing conditions.

These and other objectives of the present inventive concept are at least partly met by the invention as defined in the independent claims . Document US 2011/0288605 A1 discloses a system and a method according to the preamble of the independent claims. Preferred embodiments are set out in the dependent claims.

According to a first aspect, there is provided a system for acquiring an electrical signal, said system comprising: a plurality of electrodes, each being configured to acquire an electrode signal; a plurality of signal quality detectors, each signal quality detector being associated with at least a pair of electrodes among the plurality of electrodes, wherein each detector is configured to detect a signal based on input from the pair of electrodes and each detector comprises an analog-to-digital converter, which is configured to provide a digital representation of the detected signal, wherein the digital representation has a first resolution; a signal selection logic, which is configured to receive the digital representations determined by the detectors and is configured to determine at least one quality measure of each of the digital representations for selecting a pair of electrodes for signal acquisition and output a control signal; a multiplexer, which is connected to each of the plurality of electrodes and which is configured to select a pair of electrodes for signal acquisition based on the control signal from the signal selection logic; and a signal processing unit, which is configured to receive the selected signal from the multiplexer based on the selected pair of electrodes and which is configured to perform analog-to-digital conversion on the selected signal and to provide a digital representation of the selected signal, wherein the digital representation has a second resolution, which is higher than the first resolution.

The signal quality detectors of the system may allow detecting signals from pairs of electrodes while using a low power consumption. The signal selection logic may thus make a decision on which pair of electrodes provides a highest quality signal and only the signal from such a selected pair of electrodes need to be provided to the signal processing unit, which may determine a high resolution digital representation, such that the signal processing unit consumes more power than the signal quality detectors. The signal from the signal processing unit may then be used in analysis of the acquired electrical signal.

Thus, the system allows a low power consumption while using a large number of electrodes. A pair of electrodes among the large number of electrodes may be selectively used for detecting the electrical signals, such that effect of artifacts on the electrical signal may be reduced or avoided by selection of the pair of electrodes that provide a cleanest signal.

A number of bits used in a digital representation of an analog value may define a resolution of the conversion of the analog signal to a digital representation. Thus, the number of bits used determine a number of discrete levels that may be used for representing an analog value, such that the larger number of bits used, the smaller a distance between two discrete levels will be and hence the higher a resolution of representing the analog value will be.

Power consumption of analog-to-digital conversion is dependent on the number of bits to be used in the digital representation, such that the use of a lower resolution analog-to-digital conversion by the signal quality detectors allows the power consumption of the system to be limited.

Each signal quality detector may provide a digital representation of a detected signal based on a pair of electrodes. Thus, the signal quality detector may output a representation of the detected signal which may be used in determining a quality of the signal.

The signal selection logic may then determine a pair of electrodes which forms a high quality signal based on the input of digital representations from the signal quality detectors. However, it should be realized that signal quality detectors may provide further processing of the digital representations of the detected signals such that the determining of the at least one quality measure may be at least partly performed by the signal quality detectors before the signal selection receives the digital representations for selecting a pair of electrodes for signal acquisition.

The selected pair of electrodes may be the pair of electrodes which provides a highest quality signal based on the at least one quality measure. However, it should be realized that the pair of electrodes may alternatively or additionally be selected based on other factors. For instance, a pair of electrodes meeting a threshold of the at least one quality measure may be selected such that the first identified signal providing an acceptable quality may be selected. This implies that not all signals provided by the pairs of electrodes need to be analyzed, which may speed up a process of selecting the pair of electrodes for acquisition.

According to an embodiment, the signal quality detectors as well as the signal processing unit receive input from two electrodes. Thus, a voltage difference in the received inputs may form a detected signal.

The detected voltage may represent a bio-electrical signal, such as a signal naturally occurring in the living being, e.g. an electrocardiogram (ECG) signal, an electroencephalogram (EEG) signal, an electromyogram (EMG) signal, or an induced signal, such as a bio-impedance signal as induced based on a stimulation signal provided to a body of the living being.

According to an embodiment, each signal quality detector may comprise a differential amplifier, which is configured to amplify a difference in the provided inputs from the pair of electrodes. Further, the signal processing unit may also comprise a differential amplifier, which is configured to amplify a difference in the inputs from the selected pair of electrodes from the multiplexer.

According to an embodiment, the amplifiers of the signal quality detectors are configured to consume less power than the amplifier of the signal processing unit. This implies that the signal quality detectors may provide signals with relatively high noise. However, the amplifiers of the signal quality detectors may also have a low power consumption, which is beneficial to limit the power consumption of the system as a large number of signal quality detectors may be used. The signal processing unit on the other hand may use a low noise amplifier so as to enable a high quality signal to be provided from the signal processing unit in order to use the high quality signal as output from the system for further analysis of the acquired electrical signal.

According to an embodiment, the multiplexer is connected directly to the plurality of electrodes, such that the electrode signal by-passes the signal quality detector for being received by the multiplexer.

This implies that the signal to be provided to the signal processing unit need not be first processed by the signal quality detectors before reaching the signal processing unit. Thus, the signal processing unit may provide a high quality processing of the signal which is separate to the processing of the signal quality detectors.

According to an embodiment, the multiplexer and the signal processing unit are a first multiplexer and a first signal processing unit, and wherein the system further comprises a second multiplexer, which is connected to each of the plurality of electrodes and which is configured to select a pair of electrodes for signal acquisition based on the control signal from the signal selection logic, and a second signal processing unit, which is configured to perform analog-to-digital conversion on the selected signal, wherein the digital representation has the second resolution, wherein the signal selection logic is configured to selectively activate one of the first or the second signal processing unit upon determining that the pair of electrodes for signal acquisition is to be changed.

The use of a plurality of electrodes may allow identifying, at all times, the pair of electrodes that provides a highest quality signal. In order to ensure that the highest quality signal is acquired, the pair of electrodes used for signal acquisition may need to be frequently changed. However, initiating read-out from a pair of electrodes may be associated with a settling time in order for the read out signal to reach steady state to allow it to be reliably used. The settling time may therefore cause interruptions in the acquiring of the electrical signal, which may particularly affect the signal acquisition if the pair of electrodes being active needs to be frequently changed.

The system including a first and a second multiplexer and a first and a second signal processing unit is particularly advantageous in that interruptions in the acquired signal due to settling time of signal acquisition when the active pair of electrodes is changed may be avoided. The electrodes are connected to a first and a second multiplexer, such that signals from the electrodes may be processed by either the first or the second signal processing unit. The signal selection logic may thus activate one of the first and the second multiplexers and signal processing units such that the active signal processing unit receives the selected signal from the selected pair of electrodes. Then, when it is identified that a different pair of electrodes may provide a higher quality signal, the other multiplexer and signal processing unit may also be activated while the presently active signal processing unit continues to provide a digital representation of the selected signal. When the signal from the newly activated signal processing unit has been allowed to settle, a signal output by the system may be switched in order to continuously provide a high quality signal, while the pair of electrodes used for acquiring the signal is changed. Thereafter, the multiplexer and signal processing unit connected to a signal of low quality may be turned off. Thus, the system may be configured to continuously provide a reliable output signal to be further analyzed.

Since each of the electrodes is connected to both the first multiplexer and the second multiplexer, each of the multiplexers may freely select a pair of electrodes for signal acquisition among any electrodes in the plurality of electrodes. This implies that the system may allow switching acquisition of a signal from any pair of electrodes to any other pair of electrodes without an interruption in the acquired signal due to a settling time.

According to an embodiment, the signal selection logic is configured to compare the at least one quality measure for pairs of electrodes to the at least one quality measure for a currently selected pair of electrodes for signal acquisition for determining that the pair of electrodes for signal acquisition is to be changed.

Thus, the signal selection logic may by way of the comparison identify when the currently selected pair of electrodes is no longer providing a highest quality, such that the signal selection logic may trigger changing the pair of electrodes for acquisition.

The at least one quality measure determined by the signal selection logic may depend on the electrical signal to be acquired. Thus, for different types of electrical signals to be acquired, different characteristics may be important. Thus, the system may be pre-set for acquisition of a specific type of electrical signal and the signal selection logic may be configured to determine pre-defined quality measure(s). Alternatively, the signal selection logic may be controlled to change the quality measure(s) to be determined in order to dynamically adapt the system to acquisition of different types of signals.

For instance, the quality measure(s) to be used may differ for acquisition of an ECG signal as compared to a bio-impedance signal. For instance, quality of a QRS complex may be determined for an ECG signal, whereas this is not present in a bio-impedance signal. The quality measure(s) may thus be related to expected features in the signal to provide an indication whether the expected feature is present or prominent in the signal. According to an embodiment, the quality measure(s) may include a measure of a similarity of a shape of the signal to an expected or desired shape of the signal. However, it should also be realized that similar quality measure(s) may be used for different types of electrical signals to be acquired.

According to an embodiment, the at least one quality measure comprises a width of a frequency spectrum of the electrode signal.

Noise may span a broad frequency spectrum, whereas an electrical signal of interest may be concentrated to a narrow frequency band or a few frequencies. Thus, a small width of a frequency spectrum of a detected signal may provide an indication of a high quality signal.

According to an embodiment, the at least one quality measure comprises a noise floor value.

The noise floor value may indicate a signal value, below which signal information may not be extracted. Thus, a low noise floor value may provide an indication of a high quality signal.

According to an embodiment, the at least one quality measure comprises a value representing noise in frequency domain.

Thus, the detected signal may be converted to frequency domain and the quality measure may be determined by determining a value representing noise in the frequency domain.

It should be realized that any combination of a plurality of quality measures may be used. Also, other quality measures of the detected signal may be used, such as a signal-to-noise ratio.

According to an embodiment, the system comprises a first sensor unit and a second sensor unit, each of the first and the second sensor unit comprising a plurality of electrodes, a plurality of signal quality detectors, a signal selection logic and a multiplexer, wherein the first sensor unit and the second sensor unit are configured to select a pair of electrodes for signal acquisition of the sensor unit and wherein the first sensor unit and the second sensor unit are configured to acquire selected signals in different relations to a measurement subject, wherein the system further comprises an additional multiplexer, which is configured to receive the selected signals output by the multiplexers of the sensor units and to sequentially output selected signals from different sensor units to the signal processing unit.

This implies that the system may include at least a first and a second sensor unit, which each may be configured to determine a pair of electrodes providing a high quality signal, while using a low power consumption. Then, only the signal from a selected pair of electrodes may be provided from each sensor unit to the additional multiplexer. The signal processing unit may then sequentially receive selected signals from different sensor units.

In this way, the signal processing unit may only receive a few different signals from different sensor units. The signal processing unit may determine a best quality of the signals from the different sensor units for using a selected sensor unit in further signal acquisition. When quality of signal deteriorates, a new pair of electrodes from any of the sensor units may be selected.

Alternatively, the signal processing unit may receive time-multiplexed signals from the sensor units, such that the signal processing unit may acquire signals from all sensor units in a time-multiplexed manner. The acquired signals by the signal processing unit may then not need to be compared from a quality perspective, but any number of the signals may be used for further processing.

According to an embodiment, the system comprises a number of detectors corresponding to a number of possible combinations of pairs of electrodes among the plurality of electrodes.

This implies that each signal quality detector may be connected to a unique pair of electrodes and that, for each combination of pairs of electrodes, there is provided a signal quality detector. The system may thus enable any electrodes to be used in combination in signal acquisition.

However, the electrodes may be arranged within the system such that it may be known that some combinations of electrodes may not provide a relevant signal. Thus, there may be no point in determining a signal for such a combination of electrodes and, hence, the signal quality detectors may only be associated with relevant combination of electrodes.

According to an embodiment, each signal quality detector is statically connected to a pair of electrodes among the plurality of electrodes and wherein each of the plurality of detectors is connected to a unique pair of electrodes.

This implies that the signal quality detector may be connected to a pair of electrodes when the system is delivered. This may facilitate use of the system.

However, the placement of the electrodes in relation to a measurement subject may be defined by a user when the system is to be used. Thus, the signal quality detectors may be dynamically connected to pairs of electrodes after the electrodes have been arranged on a measurement subject. This may be especially relevant if not all combinations of pairs of electrodes are relevant to be analyzed.

According to an embodiment, the detectors are configured to detect a signal using a first sampling rate and wherein the signal processing unit is configured to detect the selected signal using a second sampling rate, which is higher than the first sampling rate.

Thus, the signal quality detectors may use a low sampling rate, which may further reduce power consumption of the signal quality detectors, whereas the signal processing unit may use a high sampling rate, which may facilitate high quality analysis of the acquired signal.

According to a second aspect, there is provided a wearable device for acquiring an electrical signal of a subject wearing the device, the wearable device comprising: a system according to the first aspect, and at least one carrier, which is configured to be worn on or around a body part of the subject, wherein the at least one carrier is configured to carry the system.

Effects and features of this second aspect are largely analogous to those described above in connection with the first aspect. Embodiments mentioned in relation to the first aspect are largely compatible with the second aspect.

The at least one carrier may be provided e.g. as a bracelet which may be worn around a wrist or an ankle of the subject or as a strap, which may be arranged around a chest of the subject. The at least one carrier may alternatively be provided as a patch, which may be configured to be attached to a body part. As a further alternative, the carrier may have a form factor allowing the carrier to be worn partly around a body part, such as the carrier being arranged as a headset, e.g. for allowing acquiring EEG signals by the electrodes.

The carrier may be a single unit in which each of the plurality of electrodes is arranged. However, according to an alternative, the wearable device may comprise several carriers carrying the system. For instance, the carriers may be arranged in a network of carriers, such as a network of patches.

Each carrier may carry a plurality of electrodes and a plurality of signal quality detectors for acquiring information enabling selection of a pair of electrodes for signal acquisition. A signal selection logic may be arranged in one of the carriers to enable controlling of selection of a pair of electrodes for signal acquisition. The carriers may be interconnected for wired communication between the carriers or may comprise communication units for wireless communication for controlling signal acquisition.

The system may be mounted on and/or in the at least one carrier. For instance, it should be realized that the electrodes may be mounted at a surface of the at least one carrier, whereas circuitry, such as the signal quality detectors, the signal selection logic, the multiplexer and the signal processing unit may be mounted within a housing defined by a carrier. It should also be realized that the components of the system may be implemented in thin film technology and that the carrier may be formed from a plurality of thin film layers for carrying and implementing the system.

Thanks to the system allowing a large number of electrodes to be selectively used for acquisition of a signal while allowing a low power consumption of the device, the wearable device may not need to be arranged in a very accurate relation to the subject in order to ensure that a high quality signal may be acquired. Thus, the wearable device may be easy to use and to properly arrange on the subject. For instance, it may not be necessary to arrange a bracelet in a tight relationship around a wrist of the subject, such that the bracelet may be comfortable to wear.

According to an embodiment, the wearable device comprises the system comprising a first and a second sensor unit described above, wherein the first sensor unit and the second sensor unit are mounted on or in different parts of the carrier, such that the first sensor unit and the second sensor unit are configured to be arranged to acquire electrode signals from different portions of the body part of the subject when the wearable device is worn.

This implies that the sensor units of the system may be arranged in relation to different portions of the body part. For instance, the sensor units may be defined in relation to different portions around a circumference of a wrist of the subject. This may allow making a first selection based on the signal quality detectors of candidates for a highest quality signal, whereas the signal processing unit may be configured to determine which of the signals from the sensor units to be used in acquiring the electrical signal, e.g. which part around a circumference of a bracelet that has a best relation to the wrist of the subject.

According to a third aspect, there is provided a method for controlling acquisition of an electrical signal; said method comprising: acquiring electrode signals by a plurality of pairs of electrodes; for each electrode signal acquired by a pair of electrodes, determining, by a signal quality detector, a digital representation of a detected signal, wherein the digital representation has a first resolution; selecting, by a signal selection logic, a pair of electrodes for signal acquisition based on a determined at least one quality measure of each of the digital representations; controlling a multiplexer to output a selected signal based on the selected pair of electrodes to a signal processing unit; performing, by the signal processing unit, analog-to-digital conversion on the selected signal and to provide a digital representation of the selected signal, wherein the digital representation has a second resolution, which is higher than the first resolution.

Effects and features of this third aspect are largely analogous to those described above in connection with the first and second aspects. Embodiments mentioned in relation to the first and second aspects are largely compatible with the third aspect.

The method allows determining of a pair of electrodes that are suitable for signal acquisition based on a limited power consumption and then processing the signal based on the selected pair of electrodes for acquiring a high quality signal.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a system for acquisition of an electrical signal according to an embodiment.
Fig. 2 is a schematic view illustrating details of a signal quality detector of the system.
Fig. 3 is a schematic view illustrating details of a signal processing unit of the system.
Fig. 4 is a flow chart illustrating a process of changing electrodes used for signal acquisition.
Fig. 5 is a schematic view of a system for acquisition of an electrical signal including a plurality of sensor units.
Fig. 6 is a schematic view of a wearable device including the system.
Fig. 7 is a flow chart of a method according to an embodiment.

### Detailed description

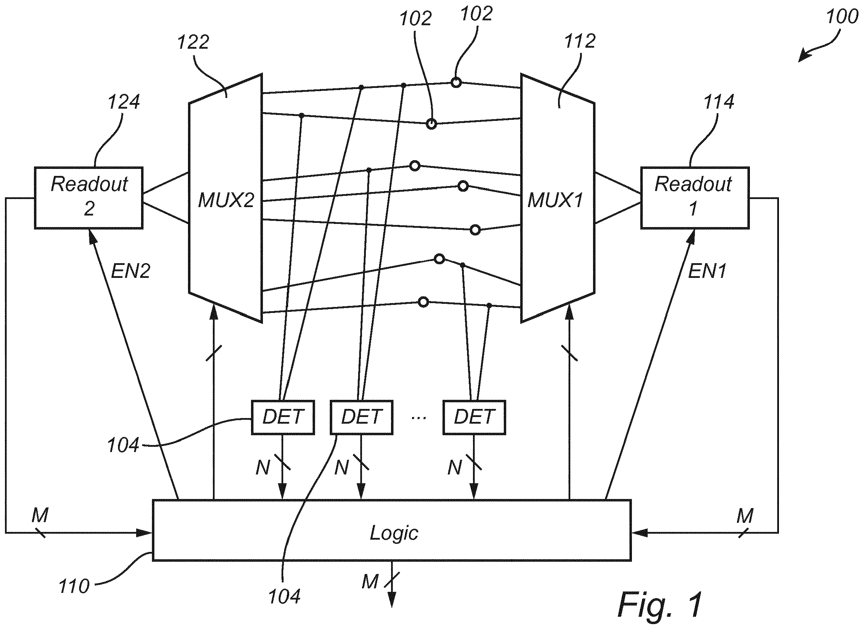

Referring now to Fig. 1, a system 100 for acquiring of an electrical signal will be described.

The system 100 comprises a plurality of electrodes 102. Each electrode 102 may be configured to be arranged in contact with a subject for acquiring an electrode signal.

The electrodes 102 may e.g. be configured for direct galvanic contact with a skin of a person. However, it should also be realized that the electrodes 102 may be arranged within a carrier, so as to be arranged in close relation to the skin of the person for forming a capacitively coupled connection to the subject.

The electrodes 102 may be designed in many different ways as will be appreciated by a person skilled in the art. For instance, the electrodes 102 may comprise an area of conductive (e.g. metallic) material for acquiring of an electrical signal. The area of conductive material may be connected to a wire for transferring the electrical signal from the electrode 102.

The system 100 further comprises a plurality of signal quality detectors 104. Each signal quality detector 104 may be connected to a pair of electrodes 102 for receiving input from the pair of electrodes 102. The signal quality detector 104 may be statically connected to a specific pair of electrodes 102 or the connection between the pair of electrodes 102 and the signal quality detector 104 may be dynamically defined.

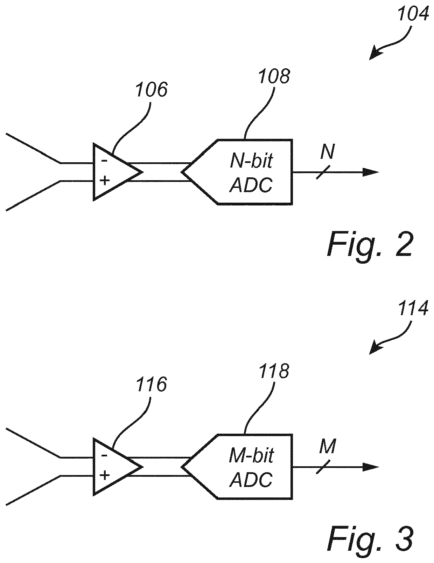

As further illustrated in Fig. 2, the signal quality detector 104 may comprise a differential amplifier 106, which is connected to receive input from the pair of electrodes 102 and amplify a difference in the signals from the electrodes 102.

The differential amplifier may provide a front-end interface of the pair of electrodes 102. The differential amplifier may be a relatively low power analog amplifier, which may provide a relatively high noise while requiring limited power consumption.

The signal quality detector 104 may further comprise an analog-to-digital converter 108, which may receive the signal from the differential amplifier 106 and which may convert the signal to a digital representation. The analog-to-digital converter 108 may be defined to provide a digital representation using N bits of data, providing a relatively low resolution of the signal detected by the signal quality detector 104 and, hence, requiring a relatively low power consumption.

The system 100 further comprises a signal selection logic 110. The signal selection logic 110 may be configured to receive the digital representations from each of the signal quality detectors 104.

The signal selection logic 110 may be configured to determine at least one quality measure of each of the digital representations. The determination of the at least one quality measure may be used for selecting a pair of electrodes 102 to be used for signal acquisition.

According to an embodiment, the signal selection logic 110 may be configured to determine which of the digital representations from the signal quality detectors 104 that corresponds to a highest quality signal based on the at least one quality measure.

However, it should be realized that the signal selection logic 110 may alternatively be configured to select the pair of electrodes 102 for signal acquisition in another manner. For instance, a threshold of an acceptable quality may be set and the signal selection logic 110 may be configured to select a pair of electrodes 102 for signal acquisition based on a first identified digital representation that exceeds the threshold. This implies that a process of selecting a pair of electrodes 102 by the signal selection logic 110 may be quickly terminated, which may reduce power consumption by the signal selection logic 110 and may ensure that the pair of electrodes 102 for signal acquisition may be quickly selected.

According to another alternative, a maximum time may be allowed for the signal selection logic 110 to select the pair of electrodes 102. Thus, the pair of electrodes 102 providing a highest quality signal as identified within the maximum time may be selected by the signal selection logic 110.

The at least one quality measure may be any of a plurality of different measures indicating quality of a signal. For instance, the at least one quality measure may be selected from a group comprising: a width of a frequency spectrum of a signal from pair of electrodes, a noise floor value, a value representing noise in frequency domain, or a signal-to-noise ratio.

According to an embodiment, the quality measure may relate to specific signal characteristics of the electrical signal to be acquired by the electrodes 102, such as characteristics of a QRS complex in an ECG signal.

The signal selection logic 110 may be implemented in hardware, or as any combination of software and hardware. The signal selection logic 110 may be implemented as software being executed on a general-purpose computer. The system 100 may thus comprise a processing unit, such as a central processing unit (CPU), which may execute the instructions of one or more computer programs in order to implement functionality of the signal selection logic 110.

The signal selection logic 110 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality of the signal selection logic 110.

The system 100 may further comprise a multiplexer 112. The multiplexer 112 may be connected to each of the plurality of electrodes 102. The multiplexer 112 may further receive a control signal from the signal selection logic 110 for controlling selection of the pair of electrodes 102.

Based on the control signal, the multiplexer 112 may provide signals from the two selected electrodes 102 to be passed to a signal processing unit 114.

In Fig. 1, the system 100 is illustrated as having a first multiplexer 112 and a second multiplexer 122, as well as a first signal processing unit 114 and a second signal processing unit 124. However, it should be realized that the system 100 may include only a single multiplexer 112 and a single signal processing unit 114, which may be used in acquiring a signal from the selected pair of electrodes 102 and processing the acquired signal.

As further illustrated in Fig. 3, the signal processing unit 114 may comprise a differential amplifier 116, which is connected to receive input from the pair of electrodes 102 as selected by the multiplexer 112 and amplify a difference in the signals from the electrodes 102.

The differential amplifier may provide a front-end interface of the pair of electrodes 102. The differential amplifier may be a relatively high power analog amplifier, which may provide a relatively low noise while requiring limited power consumption. Thus, the differential amplifier 116 of the signal processing unit 114 may have a higher power consumption than the differential amplifier 106 of the signal quality detector 104, while providing a lower noise signal than the differential amplifier 106 of the signal quality detector 104.

The signal processing unit 114 may further comprise an analog-to-digital converter 118, which may receive the signal from the differential amplifier 116 and which may convert the signal to a digital representation. The analog-to-digital converter 118 may be defined to provide a digital representation using M bits of data. The analog-to-digital converter 118 may provide a relatively high resolution of the signal and, hence, require a relatively high power consumption. The M bits of data may be higher than the N bits of data provided by the signal quality detector 104, such that the signal processing unit 114 may require a higher power consumption for the analog-to-digital conversion than the signal quality detector 104 while providing a higher resolution of the digital representation of the signal than the signal quality detector 104.

In addition, the signal quality detector 106 may possibly use a lower sampling rate than the signal processing unit 114, such that the signal quality detector 106 may further limit its power consumption.

As illustrated in Fig. 1, the signal processing unit 114 may output a digital representation of the selected signal to the signal selection logic 110. The signal selection logic 110 may then further output the signal to an external unit or another part of the system 100, wherein the signal may be e.g. further processed or may be output for display to a user. However, it should be realized that the signal processing unit 114 may directly output the digital representation of the selected signal to the external unit or another part of the system 100.

As mentioned above, the system 100 may comprise a first multiplexer 112 and a first signal processing unit 114 and a second multiplexer 122 and a second signal processing unit 124.

The signal selection logic 110 may selectively activate one of the first multiplexer 112 and first signal processing unit 114 or the second multiplexer 122 and the second signal processing unit 124. This may be used by the system 100 such that, when a change in electrodes 102 to be used for signal acquisition is to be made, an inactive multiplexer and signal processing unit may be allowed to settle before the currently active multiplexer and signal processing unit is inactivated.

Hence, since the electrodes 102 are connected to both the first multiplexer 112 and the second multiplexer 122, it is possible to freely choose the pair of electrodes 102 selected by each of the multiplexers 112, 122 to provide a signal to the first and the second signal processing unit 114, 124, respectively. Thus, when it is identified that a change in the pair of electrodes 102 being used for signal acquisition is needed, the inactive multiplexer and signal processing unit may be activated to acquire a signal using a new pair of electrodes 102 providing a signal of high quality. Then, the newly activated signal processing unit has been allowed to settle while the previously active signal processing unit continues to provide an acquired signal. Once the newly activated signal processing unit has been allowed to settle, the signal selection logic 110 may switch the signal being output by the signal selection logic 110 to use the signal received from the newly activated signal processing unit. Thus, a signal may be continuously output by the signal selection logic 110 to an external unit or another part of the system 100, without any interruptions in the signal due to a settling time of the signal processing unit.

Once the signal selection logic 110 has switched the signal being output, the previously active signal processing unit may be turned off and may be ready to be activated using a new pair of electrodes 102 if it is decided that another change of the pair of electrodes 102 being selected for signal acquisition is needed for maintaining quality of the output signal.

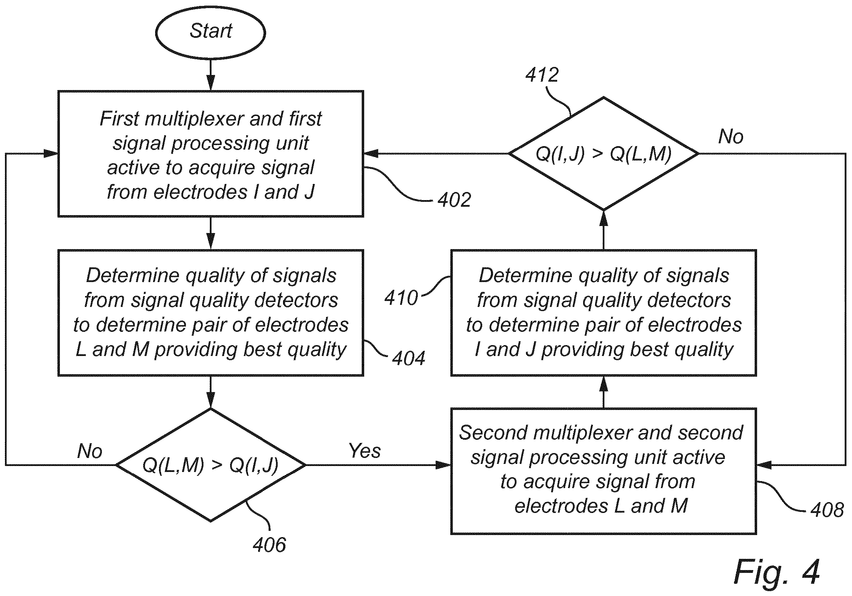

Referring now to Fig. 4, a process of changing electrodes 102 being used for signal acquisition is further described.

The process starts with the first multiplexer 112 and the first signal processing unit 114 being active, step 402. The first multiplexer 112 is configured to select a pair of electrodes 102 for signal acquisition. The selected electrodes by the first multiplexer 112 are herein denoted *i* and *j.* The signal processing unit 114 receives the signal from the electrodes *i* and *j* and processes the received signal to provide a digital representation of the signal.

While the first multiplexer 112 and the first signal processing unit 114 are active, the signal quality detectors 104 detect a signal from each pair of electrodes 102 to which the signal quality detectors 104 are connected and form digital representations of the detected signals. The signal selection logic 110 receives the digital representations from the signal quality detectors 104 and determines at least one quality measure for each of the digital representations. The signal selection logic 110 determines the pair of electrodes, herein denoted electrodes *l* and *m*, providing a best data quality based on the at least one quality measure, step 404.

Then, the quality of data Q(l,m) of the electrodes *l* and *m* is compared to the quality of data Q(i,j) of presently used electrodes *i* and *j,* step 406. If the quality of data Q(i,j) of electrodes *i* and *j* is better, the signal acquisition continues to be based on the electrodes *i* and *j* already selected by the first multiplexer 112 and the process returns to step 402. However, if the quality of data Q(l,m) of electrodes *l* and *m* is better, the pair of electrodes 102 used for signal acquisition needs to be changed and the process initiates changing of electrodes.

It should be realized that the determining of which pair of electrodes provides a best quality may be performed in many different ways. The quality of data obtained by the presently used electrodes *i* and *j*, as determined based on the digital representation from the signal quality detector 104 connected to electrodes *i* and *j,* may be compared in any order to the quality of data of the other electrode pairs. For instance, as indicated above, the best quality measure of non-selected pair of electrodes 102 may first be determined and then compared to the quality measure of the active electrodes *i* and *j,* as indicated above. According to one alternative, the quality measures may be pairwise compared, storing the best quality measure, until all quality measures have been considered, whereby the electrodes corresponding to the best quality measure is identified as the pair of electrodes providing best quality of data. If the presently used electrodes *i* and *j* provide best quality of data, the process returns to step 402; otherwise the process initiates changing of electrodes.

When electrodes selected for acquisition are to be changed, the signal selection logic 110 transmits an enable signal to the second multiplexer 122 and the second signal processing unit 124, configuring the second multiplexer 122 to select electrodes *l* and *m* for signal acquisition, step 408. The first multiplexer 112 and the first signal processing unit 122 may be turned off after the second signal processing unit 124 has been allowed to settle.

Similar to the step 404, the signal selection logic 110 receives the digital representations from the signal quality detectors 104 and determines at least one quality measure for each of the digital representations. The signal selection logic 110 determines the pair of electrodes, herein denoted electrodes *i* and *j,* providing a best data quality based on the at least one quality measure, step 410.

Then, the quality of data Q(i,j) of the electrodes *i* and *j* is compared to the quality of data Q(l,m) of presently used electrodes *l* and *m*, step 412. If the quality of data Q(l,m) of electrodes *l* and *m* is better, the signal acquisition continues to be based on the electrodes *l* and *m* already selected by the second multiplexer 122 and the process returns to step 408. However, if the quality of data Q(i,j) of electrodes *i* and *j* is better, the pair of electrodes 102 used for signal acquisition needs to be changed and the process initiates changing of electrodes, returning to step 402.

It should be realized that, although the process of changing electrodes 102 being used for signal acquisition has been described above in relation to using first and second multiplexers 112, 122 and first and second signal processing units 122, 124, a single multiplexer and a single signal processing unit may be used instead. In such case, when it is identified that the pair of electrodes 102 being used for signal acquisition is to be changed, the multiplexer may receive a control signal to control a change in the electrodes being selected by the multiplexer so as to provide a selected signal from a different pair of electrodes 102 to the signal processing unit. However, in such a set-up, interruptions in the acquired signal may occur while the signal processing unit settles when a signal from a newly selected pair of electrodes is being provided. If the pair of electrodes 102 used for signal acquisition is rarely changed, such interruptions may however be acceptable.

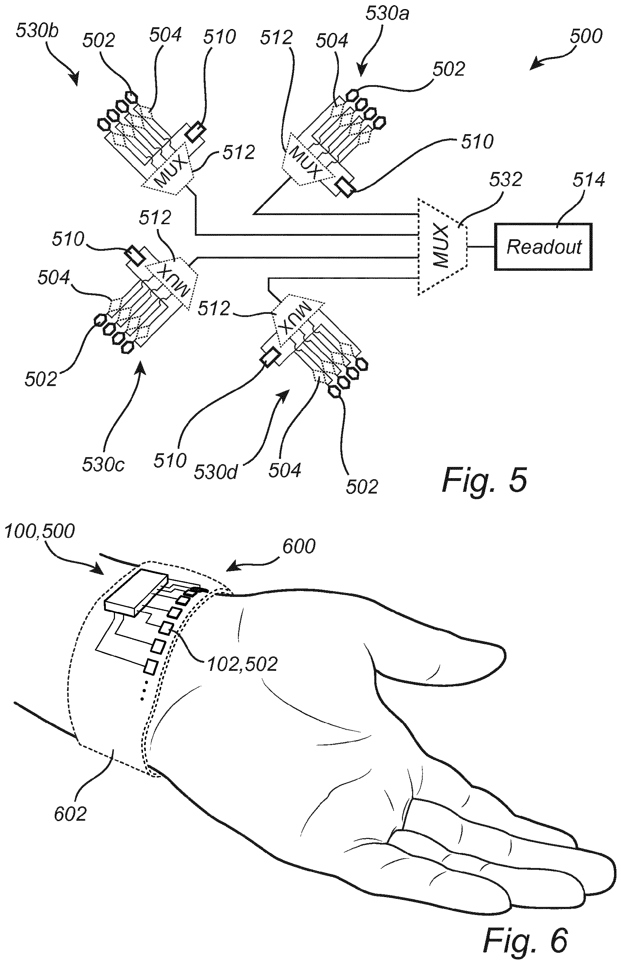

Referring now to Fig. 5, a system 500 according to another embodiment will be described.

The system 500 comprises a plurality of sensor units 530. The system 500 may comprise at least two sensor units 530, but is illustrated here as comprising four different sensor units 530a, 530b, 530c and 530d.

Each sensor unit 530a-d may comprise a plurality of electrodes 502, a plurality of signal quality detectors 504, a signal selection logic 510 and a multiplexer 512. The signal selection logic 510 and the signal quality detectors 504 of each sensor unit 530a-d may function, as described above, to determine the pair of electrodes 502 of the respective sensor unit 530a-d providing a best quality signal. The signal selection logic 510 may further control the multiplexer 512 of each sensor unit 530a-d to select the signal from the pair of electrodes 502 providing best quality for output by the multiplexer 512.

The multiplexers 512 of the sensor units 530a-d are connected to an additional multiplexer 532 of the system 500. The additional multiplexer 532 may be further configured to selectively pass the signal from a multiplexer 512 of one of the sensor units 530a-d. The additional multiplexer 532 may be connected to a signal processing unit 514 of the system 500 for providing a selected signal to the signal processing unit 514. The signal processing unit 514 may then perform analog-to-digital conversion on the selected signal and provide a digital representation of the selected signal, which may be output to an external unit or another part of the system 500, e.g. for further processing of the signal.

The plurality of sensor units 530a-d may be configured to acquire selected signals in different relations to a measurement subject. This implies that, e.g. when the measurement subject moves, the sensor unit 530a-d providing a best quality of data may change. Thanks to the signal selection logic 510 and the signal quality detectors 504 of each of the sensor units 530a-d, each sensor unit 530a-d may output the signal from the pair of electrodes 502 providing a highest quality data among the electrodes 502 of the respective sensor unit 530a-d.

With the set-up of the system 500, multiplexing may be handled in a hierarchical way. The signal processing unit 514 may only receive a few different signals from different sensor units 530a-d. The signal processing unit 514 may determine a best quality of the signals from the different sensor units 530a-d for using a selected sensor unit 530a-d in further signal acquisition. When quality of signal deteriorates, a new pair of electrodes 502 from any of the sensor units 530a-d may be selected.

Alternatively, the signal processing unit 514 may receive time-multiplexed signals from the sensor units 530a-d, such that the signal processing unit 514 may acquire signals from all sensor units 530a-d in a time-multiplexed manner. The acquired signals by the signal processing unit 514 may then not need to be compared from a quality perspective and quality of data of the signal from each of the sensor units 530a-d is ensured by the selection of the best pair of electrodes 502, as determined within each sensor unit 530a-d by the respective signal selection logic 510 and signal quality detectors 504.

Referring now to Fig. 6, a wearable device 600 is shown. The wearable device 600 may comprise at least one carrier 602, which may be worn by a subject, herein illustrated as a bracelet. The carrier 602 may further be configured to carry the system 100; 500 of any of the above-described embodiments. Thus, by the system 100; 500 being mounted on or in the carrier 602, the electrodes 102, 502 may be arranged in close proximity to the subject for acquiring an electrical signal of the subject wearing the wearable device 600.

The carrier 602 may have any shape or form facilitating the carrier 602 being worn by the subject. The carrier 602 may thus e.g. be designed as a bracelet which may be worn around a wrist or an ankle of the subject or as a strap, which may be arranged around a chest of the subject. According to further alternatives, the carrier 602 may be designed as a patch, which may comprise or be associated with an adhesive surface for attaching the patch to the subject, or as a headset for arranging the system 100, 500 on a head of the subject. The electrodes 102, 502 may be mounted on or in the carrier 602 in such manner that the electrodes 102, 502 are arranged in a suitable relation to the subject, when the carrier 602 is worn by the subject, for facilitating acquisition of the electrical signal. The electrodes 102, 502 may then be further associated with signal quality detectors, signal selection logic, multiplexer(s), and signal processing unit(s) as schematically illustrated by connections to the electrodes 102, 502 in Fig. 6.

Thanks to the system 100; 500 allowing a large number of electrodes to be selectively used for acquisition of a signal while allowing a low power consumption of the device, the wearable device 600 may not need to be arranged in a very accurate relation to the subject in order to ensure that a high quality signal may be acquired. Thus, the electrodes 102, 502 may be distributed over a large area of the carrier 602 in order to be arranged in different relations to the subject, such that at least one pair of electrodes 102, 502 may be arranged in such relation to the subject to enable acquisition of a high quality signal.

This implies that the wearable device 600 may be easy to use and to properly arrange on the subject, as it may not be necessary to arrange the wearable device 600 in a specific relation to the subject to enable acquiring of the electrical signal with high quality. For instance, it may not be necessary to arrange a bracelet in a tight relationship around a wrist of the subject, which implies that the bracelet may be comfortable to wear.

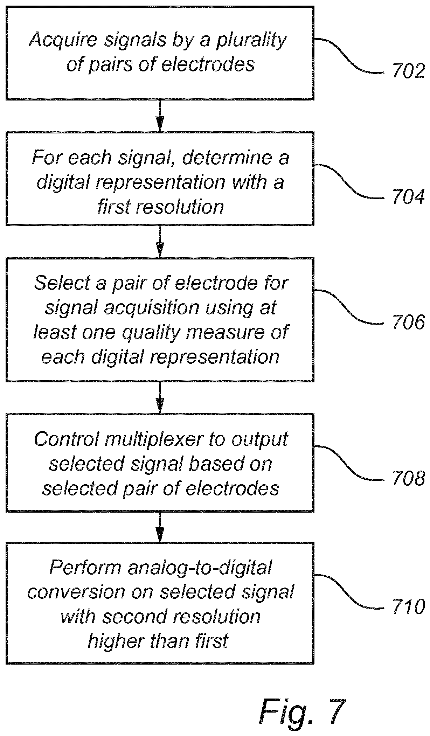

Referring now to Fig. 7, a method for controlling acquisition of an electrical signal according to an embodiment will be briefly summarized.

The method comprises acquiring, step 702, electrode signals by a plurality of pairs of electrodes. The method further comprises, for each electrode signal acquired by a pair of electrodes, determining, step 704, by a signal quality detector, a digital representation of a detected signal, wherein the digital representation has a first resolution. Thus, for each pair of electrodes, a signal is detected in order to allow a quality measure of the signal to be determined. The digital representation is determined with a first, low resolution, such that the method detects the signals of each pair of electrodes with a low power consumption.

The method further comprises selecting, step 706, by a signal selection logic, a pair of electrodes for signal acquisition. The signal selection logic may use at least one quality measure of each of the digital representations determined by the signal quality detectors and may determine a pair of electrodes that provides a highest signal quality for selecting that pair of electrodes for signal acquisition.

The method further comprises controlling, step 708, a multiplexer to output a selected signal based on the selected pair of electrodes to a signal processing unit. Thus, the signal from the selected pair of electrodes, which provides a highest signal quality, may be output by the multiplexer.

The method further comprises performing, step 710, by the signal processing unit, analog-to-digital conversion on the selected signal being output by the multiplexer. The signal processing unit may thus provide a digital representation of the selected signal, wherein the digital representation has a second resolution, which is higher than the first resolution. Thus, the relatively large power consumption associated with the analog-to-digital conversion to a high resolution digital representation is restricted to analog-to-digital conversion on the selected signal from one pair of electrodes, whereas a low power consumption is required for the analog-to-digital conversions of the plurality of signals from different pairs of electrodes used for selecting the signal to be output to the signal processing unit.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100; 500) for acquiring an electrical signal, said system comprising:
a plurality of electrodes (102; 502), each being configured to acquire an electrode signal;
a plurality of signal quality detectors (104; 504), each signal quality detector (104; 504) being associated with at least a pair of electrodes (102; 502) among the plurality of electrodes (102; 502), wherein each detector (104; 504) is configured to detect a signal based on input from the pair of electrodes (102; 502) and each detector (104; 504) comprises an analog-to-digital converter, which is configured to provide a digital representation of the detected signal, wherein the digital representation has a first resolution;
a signal selection logic (110; 510), which is configured to receive the digital representations determined by the detectors (104; 504) and is configured to determine at least one quality measure of each of the digital representations for selecting a pair of electrodes (102; 502) for signal acquisition and output a control signal;
a multiplexer (112, 122; 512, 532), which is connected to each of the plurality of electrodes (102; 502) and which is configured to select a pair of electrodes (102; 502) for signal acquisition based on the control signal from the signal selection logic (110; 510); and
a signal processing unit (114, 124; 514), which is configured to receive the selected signal from the multiplexer (112, 122; 512, 532) based on the selected pair of electrodes (102; 502) and which is configured to perform analog-to-digital conversion on the selected signal and thereby to provide a digital representation of the selected signal, **characterised in that** the digital representation has a second resolution, which is higher than the first resolution.

2. The system according to claim 1, wherein the multiplexer (112, 122; 512, 532) is connected directly to the plurality of electrodes (102; 502), such that the electrode signal by-passes the signal quality detector (104; 504) for being received by the multiplexer (112, 122; 512, 532).

3. The system according to claim 1 or 2, wherein the multiplexer and the signal processing unit are a first multiplexer (112) and a first signal processing unit (114), and wherein the system (100) further comprises a second multiplexer (122), which is connected to each of the plurality of electrodes (102) and which is configured to select a pair of electrodes (102) for signal acquisition based on the control signal from the signal selection logic (110), and a second signal processing unit (124), which is configured to perform analog-to-digital conversion on the selected signal, wherein the digital representation has the second resolution, wherein the signal selection logic (110) is configured to selectively activate one of the first or the second signal processing unit (114, 124) upon determining that the pair of electrodes (102) for signal acquisition is to be changed.

4. The system according to claim 3, wherein the signal selection logic (110) is configured to compare the at least one quality measure for pairs of electrodes (102) to the at least one quality measure for a currently selected pair of electrodes (102) for signal acquisition for determining that the pair of electrodes (102) for signal acquisition is to be changed.

5. The system according to any one of the preceding claims, wherein the at least one quality measure comprises a width of a frequency spectrum of the electrode signal.

6. The system according to any one of the preceding claims, wherein the at least one quality measure comprises a noise floor value.

7. The system according to any one of the preceding claims, wherein the at least one quality measure comprises a value representing noise in frequency domain.

8. The system according to any one of the preceding claims, wherein the system (500) comprises a first sensor unit (530a) and a second sensor unit (530b), each of the first and the second sensor unit (530a, 530b) comprising a plurality of electrodes (502), a plurality of signal quality detectors (504), a signal selection logic (510) and a multiplexer (512), wherein the first sensor unit (530a) and the second sensor unit (530b) are configured to select a pair of electrodes (502) for signal acquisition of the sensor unit (530a, 530b) and wherein the first sensor unit (530a) and the second sensor unit (530b) are configured to acquire selected signals in different relations to a measurement subject, wherein the system (500) further comprises an additional multiplexer (532), which is configured to receive the selected signals output by the multiplexers (512) of the sensor units (530a, 530b) and to sequentially output selected signals from different sensor units (530a, 530b) to the signal processing unit (514).

9. The system according to any one of the preceding claims, wherein the system (100; 500) comprises a number of detectors (104; 504) corresponding to a number of possible combinations of pairs of electrodes (102; 502) among the plurality of electrodes (102; 502).

10. The system according to claim 9, wherein each detector (104; 504) is statically connected to a pair of electrodes (102; 502) among the plurality of electrodes (102; 502) and wherein each of the plurality of detectors (104; 504) is connected to a unique pair of electrodes (102; 502).

11. The system according to any one of the preceding claims, wherein the detectors (104; 504) are configured to detect a signal using a first sampling rate and wherein the signal processing unit (114, 124; 514) is configured to detect the selected signal using a second sampling rate, which is higher than the first sampling rate.

12. A wearable device (600) for acquiring an electrical signal of a subject wearing the device (600), the wearable device (600) comprising:
a system (100; 500) according to any one of the preceding claims,
at least one carrier (602), which is configured to be worn on or around a body part of the subject, wherein the at least one carrier is configured to carry the system (100; 500).

13. The wearable device according to claim 12, comprising the system (500) according to claim 8, wherein the first sensor unit (530a) and the second sensor unit (530b) are mounted on or in different parts of the carrier (602), such that the first sensor unit (530a) and the second sensor unit (530b) are configured to be arranged to acquire electrode signals from different portions of the body part of the subject when the wearable device (600) is worn.

14. A method for controlling acquisition of an electrical signal; said method comprising:
acquiring (702) electrode signals by a plurality of pairs of electrodes (102; 502);
for each electrode signal acquired by a pair of electrodes (102; 502), determining (704), by an analog-to-digital converter comprised in a signal quality detector (104; 504) associated with said pair of electrodes, a digital representation of a detected signal, wherein the digital representation has a first resolution;
selecting (706), by a signal selection logic (110; 510), a pair of electrodes (102; 502) for signal acquisition based on a determined at least one quality measure of each of the digital representations provided by the signal quality detectors (104; 504);
controlling (708) a multiplexer (112, 122; 512, 532) to output a selected signal based on the selected pair of electrodes (102; 502) to a signal processing unit (114, 124; 514);
performing (710), by the signal processing unit (114, 124; 514), analog-to-digital conversion on the selected signal and thereby providing a digital representation of the selected signal, **characterised in that** the digital representation has a second resolution, which is higher than the first resolution.

## Patentansprüche

1. System (100; 500) zum Erfassen eines elektrischen Signals, wobei das System Folgendes umfasst:
eine Vielzahl von Elektroden (102; 502), wobei jede konfiguriert ist, um ein Elektrodensignal zu erfassen;
eine Vielzahl von Signalqualitätsdetektoren (104; 504), wobei jeder Signalqualitätsdetektor (104; 504) mit mindestens einem Paar von Elektroden (102; 502) aus der Vielzahl von Elektroden (102; 502) verknüpft ist, wobei jeder Detektor (104; 504) konfiguriert ist, um ein Signal basierend auf einer Eingabe von dem Paar von Elektroden (102; 502) zu detektieren, und jeder Detektor (104; 504) einen Analog-Digital-Wandler umfasst, der konfiguriert ist, um eine digitale Darstellung des detektierten Signals bereitzustellen, wobei die digitale Darstellung eine erste Auflösung aufweist;
eine Signalauswahllogik (110; 510), die konfiguriert ist, um die digitalen Darstellungen zu empfangen, die von den Detektoren (104; 504) bestimmt werden, und konfiguriert ist, um mindestens eine Qualitätsmessung jeder der digitalen Darstellungen zu bestimmen, um ein Paar von Elektroden (102; 502) zur Signalerfassung auszuwählen und ein Steuersignal auszugeben;
einen Multiplexer (112, 122; 512, 532), der mit jeder der Vielzahl von Elektroden (102; 502) verbunden ist, und der konfiguriert ist, um ein Paar von Elektroden (102; 502) zur Signalerfassung basierend auf dem Steuersignal von der Signalauswahllogik (110; 510) auszuwählen; und
eine Signalverarbeitungseinheit (114, 124; 514), die konfiguriert ist, um das ausgewählte Signal von dem Multiplexer (112, 122; 512, 532) basierend auf dem ausgewählten Paar von Elektroden (102; 502) zu empfangen, und die konfiguriert ist, um eine Analog-Digital-Wandlung an dem ausgewählten Signal vorzunehmen, und um dadurch eine digitale Darstellung des ausgewählten Signals bereitzustellen,
**dadurch gekennzeichnet, dass** die digitale Darstellung eine zweite Auflösung aufweist, die höher als die erste Auflösung ist.

2. System nach Anspruch 1, wobei der Multiplexer (112, 122; 512, 532) direkt mit der Vielzahl von Elektroden (102; 502) verbunden ist, so dass das Elektrodensignal den Signalqualitätsdetektor (104; 504) umgeht, um von dem Multiplexer (112, 122; 512, 532) empfangen zu werden.

3. System nach Anspruch 1 oder 2, wobei der Multiplexer und die Signalverarbeitungseinheit ein erster Multiplexer (112) und eine erste Signalverarbeitungseinheit (114) sind, und wobei das System (100) ferner einen zweiten Multiplexer (122), der mit jeder der Vielzahl von Elektroden (102) verbunden ist, und der konfiguriert ist, um ein Paar von Elektroden (102) zur Signalerfassung basierend auf dem Steuersignal von der Signalauswahllogik (110) auszuwählen, und eine zweite Signalverarbeitungseinheit (124), die konfiguriert ist, um eine Analog-Digital-Wandlung an dem ausgewählten Signal vorzunehmen, umfasst, wobei die digitale Darstellung die zweite Auflösung aufweist, wobei die Signalauswahllogik (110) konfiguriert ist, um selektiv eine von der ersten oder der zweiten Signalverarbeitungseinheit (114, 124) zu aktivieren, wenn bestimmt wird, dass das Paar von Elektroden (102) zur Signalerfassung zu ändern ist.

4. System nach Anspruch 3, wobei die Signalauswahllogik (110) konfiguriert ist, um die mindestens eine Qualitätsmessung für Paare von Elektroden (102) mit der mindestens einen Qualitätsmessung für ein derzeit ausgewähltes Paar von Elektroden (102) zur Signalerfassung zu vergleichen, um zu bestimmen, dass das Paar von Elektroden (102) zur Signalerfassung zu ändern ist.

5. System nach einem der vorhergehenden Ansprüche, wobei die mindestens eine Qualitätsmessung eine Breite eines Frequenzspektrums des Elektrodensignals umfasst.

6. System nach einem der vorhergehenden Ansprüche, wobei die mindestens eine Qualitätsmessung einen Wert des Grundrauschens umfasst.

7. System nach einem der vorhergehenden Ansprüche, wobei die mindestens eine Qualitätsmessung einen Wert umfasst, der ein Rauschen im Frequenzbereich darstellt.

8. System nach einem der vorhergehenden Ansprüche, wobei das System (500) eine erste Sensoreinheit (530a) und eine zweite Sensoreinheit (530b) umfasst, wobei jede von der ersten und der zweiten Sensoreinheit (530a, 530b) eine Vielzahl von Elektroden (502), eine Vielzahl von Signalqualitätsdetektoren (504), eine Signalauswahllogik (510) und einen Multiplexer (512) umfasst, wobei die erste Sensoreinheit (530a) und die zweite Sensoreinheit (530b) konfiguriert sind, um ein Paar von Elektroden (502) zur Signalerfassung der Sensoreinheit (530a, 530b) auszuwählen, und wobei die erste Sensoreinheit (530a) und die zweite Sensoreinheit (530b) konfiguriert sind, um ausgewählte Signale in verschiedenen Beziehungen zu einer Messperson zu erfassen, wobei das System (500) ferner einen zusätzlichen Multiplexer (532) umfasst, der konfiguriert ist, um die ausgewählten Signale zu empfangen, die von den Multiplexern (512) der Sensoreinheiten (530a, 530b) ausgegeben werden, und um der Reihe nach ausgewählte Signale von verschiedenen Sensoreinheiten (530a, 530b) an die Signalverarbeitungseinheit (514) auszugeben.

9. System nach einem der vorhergehenden Ansprüche, wobei das System (100; 500) eine Anzahl von Detektoren (104; 504) umfasst, die einer Anzahl von möglichen Kombinationen von Paaren von Elektroden (102; 502) aus der Vielzahl von Elektroden (102; 502) entspricht.

10. System nach Anspruch 9, wobei jeder Detektor (104; 504) mit einem Paar von Elektroden (102; 502) aus der Vielzahl von Elektroden (102; 502) statisch verbunden ist, und wobei jeder der Vielzahl von Detektoren (104; 504) mit einem eindeutigen Paar von Elektroden (102; 502) verbunden ist.

11. System nach einem der vorhergehenden Ansprüche, wobei die Detektoren (104; 504) konfiguriert sind, um unter Verwendung einer ersten Abtastrate ein Signal zu detektieren, und wobei die Signalverarbeitungseinheit (114, 124; 514) konfiguriert ist, um das ausgewählte Signal unter Verwendung einer zweiten Abtastrate, die höher als die erste Abtastrate ist, zu detektieren.

12. Anziehbare Vorrichtung (600) zum Erfassen eines elektrischen Signals einer Person, welche die Vorrichtung (600) trägt, wobei die anziehbare Vorrichtung (600) Folgendes umfasst:
ein System (100; 500) nach einem der vorhergehenden Ansprüche,
mindestens einen Träger (602), der konfiguriert ist, um an oder um einen Körperteil der Person getragen zu werden, wobei der mindestens eine Träger konfiguriert ist, um das System (100; 500) zu tragen.

13. Anziehbare Vorrichtung nach Anspruch 12, die das System (500) nach Anspruch 8 umfasst, wobei die erste Sensoreinheit (530a) und die zweite Sensoreinheit (530b) an oder in verschiedenen Teilen des Trägers (602) montiert sind, so dass die erste Sensoreinheit (530a) und die zweite Sensoreinheit (530b) konfiguriert sind, um Elektrodensignale von verschiedenen Abschnitten des Körperteils der Person zu erfassen, wenn die anziehbare Vorrichtung (600) getragen wird.

14. Verfahren zum Steuern der Erfassung eines elektrischen Signals; wobei das Verfahren folgende Schritte umfasst:
Erfassen (702) von Elektrodensignalen durch eine Vielzahl von Paaren von Elektroden (102; 502);
für jedes Elektrodensignal, das von einem Paar von Elektroden (102; 502) erfasst wird, Bestimmen (704), durch einen Analog-Digital-Wandler, der in einem Signalqualitätsdetektor (104; 504) enthalten ist, der mit dem Paar von Elektroden verknüpft ist, einer digitalen Darstellung eines detektierten Signals, wobei die digitale Darstellung eine erste Auflösung aufweist;
Auswählen (706), durch eine Signalauswahllogik (110; 510), eines Paars von Elektroden (102; 502) zur Signalerfassung basierend auf einer bestimmten mindestens einen Qualitätsmessung jeder der digitalen Darstellungen, die von den Signalqualitätsdetektoren (104; 504) bereitgestellt werden;
Steuern (708) eines Multiplexers (112, 122; 512, 532), um ein ausgewähltes Signal basierend auf dem ausgewählten Paar von Elektroden (102; 502) an eine Signalverarbeitungseinheit (114, 124; 514) auszugeben;
Ausführen (710), durch die Signalverarbeitungseinheit (114, 124; 514), einer Analog-Digital-Wandlung an dem ausgewählten Signal und dadurch Bereitstellen einer digitalen Darstellung des ausgewählten Signals,
**dadurch gekennzeichnet, dass** die digitale Darstellung eine zweite Auflösung aufweist, die höher als die erste Auflösung ist.

## Revendications

1. Système (100 ; 500) pour l'acquisition d'un signal électrique, ledit système comprenant :
une pluralité d'électrodes (102 ; 502), chacune étant configurée pour acquérir un signal d'électrode ;
une pluralité de détecteurs de qualité de signal (104 ; 504), chaque détecteur de qualité de signal (104 ; 504) étant associé à au moins une paire d'électrodes (102 ; 502) parmi la pluralité des électrodes (102 ; 502), dans lequel chaque détecteur (104 ; 504) est configuré pour détecter un signal sur la base d'une entrée de la paire d'électrodes (102 ; 502) et chaque détecteur (104 ; 504) comprend un convertisseur analogique-numérique configuré pour fournir une représentation numérique du signal détecté, dans lequel la représentation numérique a une première résolution ;
une logique de sélection de signal (110 ; 510) qui est configurée pour recevoir les représentations numériques déterminées par les détecteurs (104 ; 504) et est configurée pour déterminer au moins une mesure de qualité de chacune des représentations numériques pour sélectionner une paire d'électrodes (102 ; 502) pour l'acquisition de signal et la sortie d'un signal de commande ;
un multiplexeur (112, 122 ; 512, 532) qui est connecté à chacune de la pluralité des électrodes (102 ; 502) et est configuré pour sélectionner une paire d'électrodes (102 ; 502) pour l'acquisition de signal sur la base du signal de commande de la logique de sélection de signal (110 ; 510) ; et
une unité de traitement de signal (114, 124 ; 514) qui est configurée pour recevoir le signal sélectionné du multiplexeur (112, 122 ; 512, 532) sur la base de la paire d'électrodes (102 ; 502) sélectionnée et qui est configurée pour réaliser une conversion analogique-numérique du signal sélectionné et ainsi fournir une représentation numérique du signal sélectionné,
**caractérisé en ce que** la représentation numérique a une deuxième résolution supérieure à la première résolution.

2. Système selon la revendication 1, dans lequel le multiplexeur (112, 122 ; 512, 532) est directement connecté à la pluralité des électrodes (102 ; 502) de manière à ce que le signal d'électrode contourne le détecteur de qualité de signal (104 ; 504) pour être reçu par le multiplexeur (112, 122 ; 512, 532).

3. Système selon la revendication 1 ou 2, dans lequel le multiplexeur et l'unité de traitement de signal sont un premier multiplexeur (112) et une première unité de traitement de signal (114), et dans lequel le système (100) comprend en outre un deuxième multiplexeur (122) qui est connecté à chacune de la pluralité d'électrodes (102) et est configuré pour sélectionner une paire d'électrodes (102) pour l'acquisition de signal sur la base du signal de commande de la logique de sélection de signal (110), et une deuxième unité de traitement de signal (124) configurée pour réaliser une conversion analogique-numérique pour le signal sélectionné, dans lequel la représentation numérique présente la deuxième résolution, dans lequel la logique de sélection de signal (110) est configurée pour activer sélectivement l'une parmi la première ou deuxième unité de traitement de signal (114, 124) à la détermination que la paire d'électrodes (102) pour l'acquisition de signal doit être changée.

4. Système selon la revendication 3, dans lequel la logique de sélection de signal (110) est configurée pour comparer l'au moins une mesure de qualité pour des paires d'électrodes (102) avec l'au moins une mesure de qualité pour une paire d'électrodes (102) actuellement sélectionnée pour l'acquisition de signal afin de déterminer que la paire d'électrodes (102) pour l'acquisition de signal doit être changée.

5. Système selon l'une quelconque des revendications précédentes, dans lequel l'au moins une mesure de qualité comprend une largeur d'un spectre de fréquences du signal d'électrode.

6. Système selon l'une quelconque des revendications précédentes, dans lequel l'au moins une mesure de qualité comprend une valeur de bruit de fond plancher.

7. Système selon l'une quelconque des revendications précédentes, dans lequel l'au moins une mesure de qualité comprend une valeur représentant un bruit de fond dans un domaine fréquentiel.

8. Système selon l'une quelconque des revendications précédentes, dans lequel le système (500) comprend une première unité de détection (530a) et une deuxième unité de détection (530b), chacune parmi la première et la deuxième unité de détection (530a, 530b) comprenant une pluralité d'électrodes (502), une pluralité de détecteurs de qualité de signal (504), une logique de sélection de signal (510) et un multiplexeur (512), dans lequel la première unité de détection (530a) et la deuxième unité de détection (530b) sont configurées pour sélectionner une paire d'électrodes (502) pour l'acquisition de signal de l'unité de détection (530a, 530b) et dans lequel la première unité de détection (530a) et la deuxième unité de détection (530b) sont configurées pour acquérir des signaux sélectionnés en relations différentes avec un sujet de mesure, dans lequel le système (500) comprend en outre un multiplexeur additionnel (532) qui est configuré pour recevoir les signaux sélectionnés qu'ont fait sortir les multiplexeurs (512) des unités de détection (530a, 530b) et pour faire sortir sélectivement des signaux sélectionnés à partir de différentes unités de détection (530a, 530b) à l'attention de l'unité de traitement de signal (514).

9. Système selon l'une quelconque des revendications précédentes, dans lequel le système (100 ; 500) comprend un certain nombre de détecteurs (104 ; 504) correspondant à un certain nombre de combinaisons possibles de paires d'électrodes (102 ; 502) parmi la pluralité d'électrodes (102 ; 502).

10. Système selon la revendication 9, dans lequel chaque détecteur (104 ; 504) est connecté de manière statique à une paire d'électrodes (102 ; 502) parmi la pluralité d'électrodes (102 ; 502) et dans lequel chacun de la pluralité des détecteurs (104 ; 504) est connecté à une unique paire d'électrodes (102 ; 502).

11. Système selon l'une quelconque des revendications précédentes, dans lequel les détecteurs (104 ; 504) sont configurés pour détecter un signal en utilisant un premier taux d'échantillonnage et dans lequel l'unité de traitement de signal (114, 124 ; 514) est configurée pour détecter le signal sélectionné en utilisant un deuxième taux d'échantillonnage qui est supérieur au premier taux d'échantillonnage.

12. Dispositif pouvant être porté (600), pour acquérir un signal électrique d'un sujet portant le dispositif (600), le dispositif pouvant être porté (600) comprenant :
un système (100 ; 500) selon l'une quelconque des revendications précédentes,
au moins un support (602) qui est configuré pour être porté sur ou autour d'une partie de corps du sujet, dans lequel l'au moins un support est configuré pour porter le système (100 ; 500).

13. Dispositif pouvant être porté selon la revendication 12, comprenant le système (500) selon la revendication 8, dans lequel la première unité de détection (530a) et la deuxième unité de détection (530b) sont montées sur ou dans différentes parties du support (602) de manière à ce que la première unité de détection (530a) et la deuxième unité de détection (530b) soient configurées pour être agencées pour acquérir des signaux d'électrode de différentes parties de la partie de corps du sujet lorsque le dispositif pouvant être porté (600) est porté.

14. Procédé pour contrôler l'acquisition d'un signal électrique ; ledit procédé comprenant :
l'acquisition (702) de signaux d'électrode par une pluralité de paires d'électrodes (102 ; 502) ;
pour chaque signal d'électrode acquis par une paire d'électrodes (102 ; 502), la détermination (704), par un convertisseur analogique-numérique compris dans un détecteur de qualité de signal (104 ; 504) associé à ladite paire d'électrodes, d'une représentation numérique d'un signal détecté, dans lequel la représentation numérique a une première résolution ;
la sélection (706), par une logique de sélection de signal (110 ; 510), d'une paire d'électrodes (102 ; 502) pour l'acquisition de signal sur la base d'au moins une mesure de qualité déterminée de chacune des représentations numériques fournies par les détecteurs de qualité de signal (104 ; 504) ;
le contrôle (708) d'un multiplexeur (112, 122 ; 512, 532) pour faire sortir un signal sélectionné sur la base de la paire d'électrodes (102 ; 502) sélectionnée à l'attention d'une unité de traitement de signal (114, 124 ; 514) ;
la réalisation (710), par l'unité de traitement de signal (114, 124 ; 514), d'une conversion analogique-numérique pour le signal sélectionné et la fourniture de la sorte d'une représentation numérique du signal sélectionné,
**caractérisé en ce que** la représentation numérique a une deuxième résolution qui est supérieure à la première résolution.
